# EUROPÄISCHE PATENTSCHRIFT

(11) **EP 1 521 522 B1**
(45) Veröffentlichungstag und Bekanntmachung des Hinweises auf die Patenterteilung: **24.08.2011**
(21) Anmeldenummer: 03763729.5
(22) Anmeldetag: 07.07.2003
(51) Int. Cl.: A01N 25/16, A01N 25/24

(54) **BEHANDLUNG VON ZITZEN ODER EUTERN VON SÄUGETIEREN NACH DEM MELKEN MIT BARRIEREBILDENDEN SCHAUMSYSTEMEN**
TREATMENT OF TEATS OR UDDERS OF MAMMALS AFTER MILKING WITH BARRIER-FORMING FOAM SYSTEMS
TRAITEMENT DE TRAYONS OU DE PIS DE MAMMIFERES, APRES LA TRAITE, AVEC DES SYSTEMES MOUSSANTS GENERATEURS DE BARRIERE

(30) Priorität: 16.07.2002 DE 10232065
(43) Veröffentlichungstag der Anmeldung: 13.04.2005
(73) Patentinhaber: ECOLAB INC., St. Paul, MN 55102-2233 (US)
(72) Erfinder: COLLIN, Alain, F-94230 Cachan (FR)
(74) Vertreter: Polypatent
(86) Internationale Anmeldenummer: PCT/EP2003/007252
(87) Internationale Veröffentlichungsnummer: WO 2004/006668

(56) Entgegenhaltungen:
- WO-A-01/22975
- WO-A-98/04136

## Beschreibung

Die vorliegende Erfindung betrifft ein nichttherapeutisches Verfahren zur Desinfektion von Zitzen oder Eutern von Säugetieren, wobei die Zitzen oder Euter nach dem Melkvorgang mit einer in Form eines Schaumes vorliegenden desinfizierenden Zusammensetzung behandelt werden. Die erfindungsgemäße Schaumzusammensetzung enthält eine filmbildende Komponente, ein Desinfektionsmittel und ein oder mehrere Tenside als Schaumbildner.

Rinder, Schafe und Ziegen werden heute überwiegend durch Anwendung automatischer Melktechniken gemolken. Die durch lange Züchtungserfolge auf hohe Milchproduktion getrimmten Tiere sind im Bereich der Milchdrüsen und Euter sehr infektionsanfällig. Die Infektionsanfälligkeit wird durch starke Beanspruchung der Zitzen während des Melkens sowie durch ständig auf die Zitzen einwirkende Umgebungsfaktoren, wie Wind, Regen und Sonnenstrahlung erhöht. Durch diese ständige Belastung der Zitzen kommt es bei den Tieren häufig zur Euterentzündung, die als "subklinische" oder "klinische Mastitis" in der Literatur beschrieben wird. Je nach Stärke der Infektion der erkrankten Euterviertel kommt es zu einer geringen bis drastischen Erhöhung somatischer Zellen in der Milch. Die Erhöhung somatischer Zellen in der Milch über das Maß gesunder Euter (200 000 Zellen pro ml) hinaus ist unerwünscht, da sie die Qualität der Milch für die weiteren Verarbeitungsschritte einschränkt und die Milch im Extremfall ungenießbar werden lässt. Eine drastische Erhöhung somatischer Zellen in der Milch geht oft mit Fieber (klinische Mastitis) einher, bis zum Verlust des Tieres durch Notschlachten. Eine Ursache für solche Infektionen ist die Einwanderung von euterpathogenen Keimen, wie z.B. Pseudomonas aeruginosa, Escherichia coli, Streptococcus uberis, Streptococcus dysgalactiae, Streptococcus agalactiae, Staphylococcus aureus. Die größte Gefahr der Euterinfektion besteht unmittelbar nach dem Melkvorgang. Dann ist der Strichkanal der Zitzen geöffnet, wodurch es den Keimen aus der Umgebung des Euters möglich ist, in den Strichkanal einzuwandern und Infektionen auszulösen.

Zur Vermeidung bzw. Verringerung des Infektionsrisikos werden die Zitzen der Tiere entweder vor dem Melken oder aber nach dem Melken behandelt.

Werden die Zitzen oder Euter der Säugetiere vor dem Melken behandelt, ergibt sich das Problem, dass die desinfizierenden bzw. reinigenden Wirkstoffe nach der Anwendung nicht in die Milch gelangen dürfen. Falls solche Substanzen in die Milch gelangen, wäre dies zum einen lebensmittelrechtlich bedenklich und zum anderen könnte z.B. die Käseherstellung durch die Anwesenheit solcher Substanzen inhibiert werden. Die EP 0 423 922 A1 beschreibt ein Verfahren, bei dem eine antimikrobielle Zusammensetzung vor dem Melken auf die Zitzen oder Euter aufgebracht wird, die nach der Applikation keine Rückstände hinterlässt. Die Reinigungszusammensetzung umfasst eine desinfizierende Komponente erhältlich aus Milchsäure und Wasserstoffperoxid. Nachdem die desinfizierende Komponente ihre desinfizierende Wirkung entfaltet hat, zerfällt sie in lebensmittelrechtlich und toxikologisch unbedenklichen Substanzen, nämlich Wasser und Milchsäure.

In EP 0 772 973 A1 und WO-A-01/22975 wird die Applikation von angepassten desinfizierenden Mischungen zur Reinigung und Desinfektion der Zitzen in Form eines Schaumes beschrieben. Die Applikation als Schaum hat den Vorteil, dass der Melker, bevor das Melkgeschirr angelegt wird, leichter feststellen kann, ob die Reinigungsflüssigkeit vollständig von dem Euter bzw. den Zitzen entfernt wurde. Üblicherweise werden die Reinigungsflüssigkeiten in einen Behälter gegeben und die Zitzen der Säugetiere werden in diesen Behälter eingetaucht. Wird nun in diesen Behälter Schaum anstatt einer Flüssigkeit gegeben, so steigt der Schaum im Vergleich zur Flüssigkeit höher, wenn Zitzen in den Behälter eingetaucht werden. Die Reinigung und Desinfektion der Zitzen wird so verbessert.

Die FR 88 08658 A1 offenbart als Reinigungsmittel und Desinfektionsmittel eine Zusammensetzung auf der Basis von aus tertiären Aminen mit hohem Molekulargewicht abgeleiteten Aminoxiden und von 1-Coco alkylguanidin einzusetzen und diese Zusammensetzung beim Desinfektionsvorgang im reinen Zustand und beim Reinigungsvorgang in einer zwischen 200 und 500-fach verdünnten Zubereitung zu verwenden. Wenn diese Zusammensetzung nach dem Melken aufgebracht werden soll, so wird erwähnt, sie als Schaum zu applizieren, da der Schaum eine höhere Kontaktzeit auf der Haut der Tiere haben soll, als das vergleichbare flüssige Produkt. In der Zubereitung ist keine Komponente enthalten, die den Strichkanal der Zitzen verschließt. Deshalb können durch das Desinfektionsmittel nicht abgetötete Keime in den geöffneten Strichkanal einwandern und eine Mastitis auslösen.

In WO 98/04/36 A1 wird ein anderer Weg zur Verhinderung von Infektionen nach dem Melkvorgang gewählt. Der nach dem Melkvorgang weit geöffnete Strichkanal der Zitzen wird durch Aufbringen eines filmbildenden Mittels, das eine filmbildende Komponente und desinfizierende Wirkstoffe enthält, verschlossen. Es zeigt sich, dass die filmbildenden Komponenten dieser Zusammensetzung speziell ausgewählt werden müssen, da an den Film, der sich auf den Zitzen bzw. den Eutern der Säugetieren nach Anwendung bildet, spezielle Anforderungen zu stellen sind. Zum einen muss der gebildete Film fest anhaftend sein und darf sich nicht durch z.B. das Liegen der Kühe auf Gras oder ähnlichem zu leicht abscheuern lassen, zum andern muss der Film aber vor dem nächsten Melkvorgang sicher entfernt werden können, da er sonst in die gemolkene Milch gelangen könnte. Die Kombination speziell ausgewählter Polyvinylalkoholpolymere oder Copolymere mit speziell ausgewählten Ethercarbonsäuren brachte den gewünschten Erfolg. Als desinfizierender Wirkstoff ließ sich Jod in Form von Polyvinylpyrrolidon-Iod und/oder Chlorhexidin einsetzen. Die Verwendung des filmbildenden Mittels erfolgt in der Art, dass man die Zitzen oder Euter von Säugetieren nach dem Melken in das Mittel eintaucht, bestreicht oder besprüht. Es bildet sich sodann ein Film, der den nach dem Melkvorgang noch geöffneten Strichkanal sicher und ohne Fehlstellen verschließt. Durch die gewählten Applikationsmethoden ist die Menge an filmbildenden Mitteln, die auf - dem Euter bzw. den Sitzen verbleibt, relativ hoch. Dies führt dazu, dass die Entfernung des Films vor dem nächsten Melkvorgang aufwendig ist. Zusätzlich dauert, bedingt durch die hohe Menge die appliziert wird, die Trocknung auf den Zitzen bzw. dem Euter der Säugetiere sehr lange. Dies führt dazu, dass die Saugetiere nach dem Melken durch Hinlegen auf z.B. eine Wiese den noch nicht getrockneten Film abreiben können. Ein weiterer Nachteil ist, dass eine Kontrolle der Menge an appliziertem Mittel durch die Transparenz des Mittels nur schwer möglich ist.

WO 01/22975 A1 beschreibt eine hautreinigende desinfizierende Schaumapplikation ohne filmbildendes Mittel, die als desinfizierende Komponenten Chlorhexidin und iodhaltige Mittel enthält.

Die Aufgabe der vorliegenden Erfindung ist es, den Strichkanal der Zitzen von Säugetieren nach dem Melken sicher zu verschließen und vorhandene Keime auf Zitzen und Euter abzutöten. Es soll vor allem die Menge an applizierter filmbildender Komponente verringert werden, so dass der sich bildende Film vor dem nächsten Melken sicher und leicht entfernt werden kann. Zusätzlich soll die Trocknungsdauer des applizierten Mittels verringert werden, so dass ein fest anhaftender Film unverzüglich ausgebildet wird, so dass er nicht mehr aus Versehen durch das Säugetier abgerieben werden kann. Weiterhin soll eine bessere Kontrolle der aufgebrachte Menge des Mittels ermöglicht werden.

Die erfindungsgemäße Aufgabe wird gelöst durch ein nichttherapeutisches Verfahren zur Desinfektion von Zitzen oder Eutern von Säugetieren, wobei die Zitzen oder Euter nach dem Melkvorgang mit einer in Form eines Schaumes vorliegenden desinfizierenden Zusammensetzung behandelt werden, die folgende Bestandteile enthält :
a) ein Polyvinylalkohol-Polymer und/oder Copolymer
b) eine Ethercarbonsäure, die vorzugsweise unter die allgemeine Formel R-(OC₂H₄)ₙ-OCH₂-COOH fällt, wobei R für einen Alkyrest mit 4 bis 10 C-Atomen und n für eine ganze Zahl im Bereich von 2 bis 8 steht als filmbildendes Mittel,
c) einen Desinfektionswirkstoff, und
d) ein oder mehrere Tenside als Schaumbildner und
e) 30 - 90 vol.-% eines Gases.

Der genannte Desinfektionswirkstoff ist vorzugsweise ausgewählt aus der Gruppe Polyvinylpyrrolidin-Jod, Chlorhexidin, Derivate von Chlorhexidin, Gemische aus Milchsäure und Wasserstoffperoxid oder Mischungen derselben.

Die Funktionsweise des erfindungsgemäßen Verfahrens besteht üblicherweise darin, daß der applizierte Schaum trocknet und sich ein geschlossener Film auf Zitzen oder Euter ausbildet. Durch den Film wird der Strichkanal der Zitzen verschlossen. Der Desinfektionswirkstoff tötet vorhandene Keime auf Zitzen oder Euter ab.

Die Behandlung der Zitzen oder Euter von Säugetieren mit der desinfizierenden filmbildenden Zusammensetzung, die als Schaum vorliegt, kann in der Gestalt erfolgen, dass der Schaum zunächst in einen Behälter gefüllt wird und dann die Zitzen oder Euter in diesen Behälter getaucht werden:

Die desinfizierende filmbildende Zusammensetzung enthält bevorzugt 1 bis 10 Gew.-% Polyvinylalkohol-Polymer und/oder Copolymer, 0,2 bis 5 Gew.-% einer Ethercarbonsäure der allgemeinen Formel R-(OC₂H₄)ₙ-OCH₂-COOH, wobei R für einen Alkylrest mit 4 bis 10 C-Atomen und n für eine ganze Zahl im Bereich von 2 bis 8 steht, als filmbildendes Mittel, 0,01 bis 5 Gew.-% Desinfektionswirkstoff und 0,1 bis 10 Gew.-% Tenside.

Die eingesetzten Polyvinylalkoholpolymere oder Copolymere haben eine Molmasse im Bereich von 18.000 bis 200.000 D, wobei 30.000 bis 180.000 D bevorzugt sind und 40.000 bis 150.000 D besonders bevorzugt sind.

Als Desinfektionswirkstoff kann 0,02 bis 0,3 Gew.-% Jod in Form von Polyvinylpyrrolidon-Jod eingesetzt werden. Es ist bevorzugt, dass 0,03 bis 0,2 Gew.-% und besonders bevorzugt 0,04 bis 0,15 Gew.% Jod in Form von Polyvinylpyrrolidin-Jod eingesetzt werden. Als Desinfektionswirkstoff können aber alternativ auch andere Desinfektionskomponenten zum Einsatz kommen, wie beispielsweise 0,05 bis 0,5 Gew.-% Chlorhexidin. Dabei ist es, wenn 0,07 bis 0,4 Gew.-%, besonders bevorzugt 0,1 bis 0,3 Gew.-% Chlorhexidin eingesetzt werden.

Die in dem Verfahren eingesetzten Tenside können anionische, kationische oder nichtionische Tenside sein.
Beispielsweise können die genannten Tenside ausgewählt sein aus den tertiären Aminoxiden der allgemeinen Formel (A) 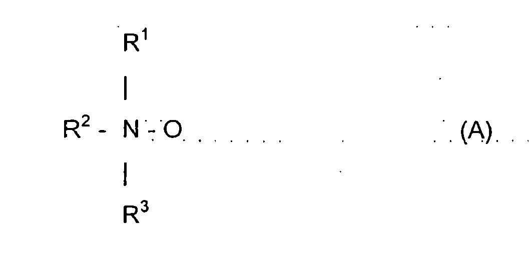 wobei R¹ eine gesättigte oder ein- bis dreifach ungesättigte verzweigte oder unverzweigte Alkylgruppe mit 10 bis 20 C-Atomen und R² und R³ unabhängig voneinander Methyl-, Ethyl-, Propylreste oder deren Hydroxyderivate bedeuten und/oder den Alkylpolyglykosiden der allgemeinen Formel (B) 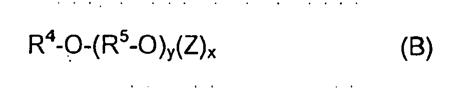 wobei R⁴ ein 6 bis 30 C-Atome aufweisender einwertiger gesättigter oder ungesättigter Alkyl- oder Hydroxyalkyl- oder ein Arylrest, R⁵ ein zweiwertiger Kohlenwasserstoffrest mit 2 bis 4 C-Atomen, y eine Zahl zwischen 0 und 12, Z ein Zuckerrest mit 5 oder 6 C-Atomen und x eine Zahl zwischen 1 und 10 bedeuten, und/oder den quartären Ammoniumverbindungen der allgemeinen Formel C 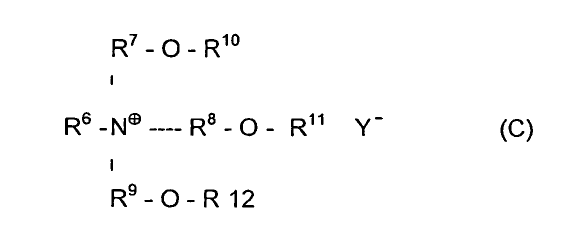 in der die einzelnen Teile unabhängig voneinander folgende Bedeutung haben:
- R⁶: = Alkyl-, Alkenyl oder Arylgruppe mit 1 bis 6 C-Atomen
- R⁷ R⁸, R⁹: = Alkylengruppe mit jeweils 2 bis 4 C-Atomen
- R¹⁰, R¹¹, R¹²: = Wasserstoff oder aliphatische oder aromatische Acylgruppe mit 6 bis 20, C-Atomen, wobei höchstens 2 der Reste R⁵ bis R⁷ Wasserstoff sein können,
- Y⁻: = anorganisches oder organisches Anion

Besonders bevorzugte Aminoxide sind die, bei denen die Substituenten R² und R³ 2-Hydroxyethylreste bedeuten Beispiele solcher Aminoxide sind Talgfett-bis-(2-hydroxyethyl-)-aminoxid, Oleyl-bis-(2-hydroxyethyl)-aminoxid, Kokos-bis-(2-hydroxyethyl-)-aminoxid.

Weitere bevorzugte Aminoxide in diesem Zusammenhang sind Tetradecyldimethyl-aminoxid und/oder andere Alkyldimethyl-aminoxide, die 12 bis 18 Kohlenstoffatome in der Alkylkette aufweisen, wie zum Beispiel Lauryldimethylaminoxid oder Myristyldimethylaminoxid.

Zu den ebenfalls bevorzugt enthaltenen Tensiden der nichtionischen Tensidklasse gehören auch die Alkylpolyglykoside. Insbesondere sind diejenigen zu erwähnen, deren Alkylgruppen aus nativen Fetten, Ölen oder petrochemisch hergestellten Alkoholen und deren Zuckerreste aus hydrolytisch gespaltenen Polysacchariden stammen. Die Alkylpolyglykoside stellen Veretherungsprodukte von Fettalkoholen fettchemischen oder petrochemischen Ursprungs mit Mono- oder Oligosacchariden dar, wobei die Zuckerreste vor der Veretherung mit den Fettalkoholen zusätzlich alkoxyliert werden können. Man erhält hierdurch Alkylpolyglykoside, die beispielsweise in der WO 86/05199 näher beschrieben sind. Technische Alkylpolyglykoside sind in der Regel keine molekular einheitlichen Produkte, sondern stellen Alkylether von Gemischen aus Mono- und unterschiedlichen Oligosacchariden dar. Besonders bevorzugt werden im Rahmen der vorliegenden Erfindung solche Alkylpolyglykoside, kurz auch als APG bezeichnet, die auf nichtethoxylierten Zuckern beruhen. Als Zuckerrest wird vorzugsweise ein Glucoserest verwendet, der als einzelne Glucoseeinheit oder als Oligoglucoseeinheit mit bis zu etwa 5 Glucosegruppen vorliegt. Der Alkylrest steht vorzugsweise für einen gesättigten oder ungesättigten Alkylrest mit 8 bis 16 C-Atomen, vorzugsweise mit 8 bis 10 C-Atomen, oder Gemischen hiervon.

Als Vertreter der genannten kationischen Tenside sind bevorzugt quartäre Ammoniumsalze (QAV), insbesondere solche gesättigte oder ungesättigte quartäre Ammoniumsalze, die aus der Veresterung von Trialkanolamin, vorzugsweise Triethanolamin, mit Fettsäuren und nachfolgender Quaternierung mit geeigneten Alkylierungsmitteln entstehen. Als Fettsäuren seien insbesondere solche mit 12 bis 18 C-Atomen, beispielsweise Laurinsäure, Myristinsäure, Palmitinsäure, Ölsäure oder Stearinsäure genannt, wobei bevorzugt die technisch anfallenden Gemische der Fettsäuren beispielsweise die von Kokos-, Palmkern-, Raps- oder Talgfett abgeleiteten Säuregemische verwendet werden. Pro Molekül enthalten diese sogenannten Esterquats im Mittel 1 bis 3 Estergruppen, wobei vorzugsweise im Mittel wenigstens 2 Estergruppen enthalten sind. Als Gegenionen enthalten die Esterquats vorzugsweise Halogenid, insbesondere Chlorid, Sulfat, Methylsulfat, Methylphosphat und Alkyl- oder Arylsulfonat.
Aufgrund der schäumenden Wirkung von quartären Ammoniumsalzen kann es aber auch bevorzugt sein, als kationisches Tensid übliche, im Markt verfügbare QAV's zu verwenden.

Es ist allerdings genauso bevorzugt, dass als die genannten Tenside solche eingesetzt werden, die ausgewählt sind aus der Gruppe Alkylsulfate, Alkylsulfonate, Alkylbenzolsulfonate, Fettalkoholethoxylate oder Mischungen derselben.

Üblicherweise werden gute Ergebnisse erreicht, wenn der Anteil der genannten Tenside bei 3 bis 10 Gew.-% Tensid, bezogen auf die gesamte Zusammensetzung, liegt.

Der Schaum kann mit allen dem Fachmann bekannten Verfahren des Standes der Technik erzeugt werden. Es ist bevorzugt, dass der Schaum durch Einblasen eines Gases in eine Zusammensetzung, die filmbildendes Mittel, Desinfektionsmittel und Tensid enthält gebildet wird. Es bildet sich ein stabiler Schaum, der sich sicher in dem erfindungsgemäßen Verfahren einsetzen lässt.

Mit dem erfindungsgemäßen Verfahren können die Zitzen und Euter aller Säugetiere behandelt werden, wobei als Säugetiere Rinder, Schafe und Ziegen besonders bevorzugt sind.

Die Schaumzusammensetzung zur Desinfektion von Zitzen oder Eutern von Säugetieren enthält:
a) 1 bis 10 Gew-% Polyvinylalkohol-Polymer oder Copolymer mit Molmassen im Bereich von 18.000 bis 200.000 D,
b) 0,2 bis 5 Gew-% einer Ethercarbonsäure der allgemeinen Formel R-(OC₂H₄)ₙ-OCH₂-COOH, wobei R für einen Alkylrest mit 4 bis 10 C-Atomen und n für eine ganze Zahl im Bereich von 2 bis 8 steht, als filmbildende Mittel,
c) 0,02 bis 0,3 Gew-% Jod in Form von Polyvinylpyrrolidon-Jod oder 0,05 bis 0,5 Gew-% Chlorhexidin als Desinfektionswirkstoff und
d) 3 bis 10 Gew-% eines Tensids.
e) 30 bis 90 vol.-% eines Gases Bevorzugt

Die Schaumzusammensetzung enthält 40 bis 80 Vol.-% und besonders bevorzugt 50 bis 70 Vol.-% eines Gases.

Es ist bevorzugt, dass die Schaumzusammensetzung eine wässrige Schaumzusammensetzung ist.

Die Schaumzusammensetzung wird in einem Verfahren zur Desinfektion von Zitzen oder Eutern von Säugetieren, wobei die Zitzen oder Euter nach dem Melkvorgang mit einer in Form eines Schaumes vorliegenden desinfizierenden Zusammensetzung behandelt werden, eingesetzt. Diese Schaumzusammensetzung führt zu einem sicheren Verschluss des Strichkanals der Zitzen nach dem Melken und zu einer sicheren Desinfektion von Zitzen und Euter. Dies ist umso überraschender, da durch das Gas, das der Schaum enthält zu erwarten wäre, dass der sich bildende Film mit Poren durchsetzt ist, so dass ein sicherer Verschluss des Strichkanals der Zitzen nicht gewährleistet wäre Die applizierte Menge ist im Vergleich zur Aufbringung durch Eintauchen der Zitzen in eine Flüssigkeit oder Einstreichen der Zitzen mit einer Flüssigkeit verringert.

Deshalb lässt sich der auf den Zitzen oder Eutern gebildete Film vor dem nächsten Melkvorgang sicher und leicht entfernen. Durch die Applikation als Schaum ist es dem Melker möglich die aufgebrachte Menge leicht zu kontrollieren.

Die vorliegende Erfindung soll an den folgenden Beispielen erläutert werden.

### Beispiele

Die Zitzen und Euter von Kühen werden nach dem Melken mit der erfindungsgemäßen Schaumzusammensetzung 1 und als Vergleich mit der Zusammensetzung 2 behandelt.

**Tabelle 1:**

| | Zusammensetzung 1 [Gew.-%] | Zusammensetzung 2 (Vergleich) [Gew.-%] |
|---|---|---|
| voll entsalztes Wasser | 83,5 | 88,5 |
| Alkylbenzol-sulfonsäure | 5,0 | 0 |
| Polyvinylalkohol-copolymer | 4,0 | 4,0 |
| Xanthan-Gum | 0,45 | 0,45 |
| Glycerol | 5,05 | 5,05 |
| Polyvinylpyrrolidon-Jod (10% Jod) | 1,0 | 1,0 |
| Caprylethercarbonsäure | 1,0 | 1,0 |
| Vol.-% Gas in der Zusammensetzung | 70 | 0 |

Die Zusammensetzung 1 bzw. 2 wurde in Behälter gegeben. Die Zitzen der Kühe wurden nach dem Melken in die entsprechenden Behälter eingetaucht. Die erfindungsgemäße Zusammensetzung 1 wurde bei 649 Kühen nach dem Melkvorgang angewendet. Zur Desinfizierung der 649 Kühe wurde insgesamt eine Menge von 2514 g der Zusammensetzung 1 verbraucht, was einen Verbrauch von 3,87 g pro Kuh entspricht. Als Vergleich wurde die Zusammensetzung 2 bei 505 Kühen nach dem Melkvorgang appliziert. Hierbei trat ein Verbrauch von 2854 g der Zusammensetzung 2 auf. Das entspricht einem Verbrauch von 5,65 g pro Kuh und Melkvorgang.

Durch Vergleich der notwendigen Mengen an desinfizierender Zusammensetzung nach dem Melkvorgang wird ersichtlich, dass die erfindungsgemäße Schaumzusammensetzung eine Ersparnis von ca. 31,5 % der benötigten Menge bewirkt. Bei der Verwendung der erfindungsgemäßen Schaumzusammensetzung 1 bildete sich ein Film, der den Strichkanal der Zitzen auf sichere Art und Weise verschloss. Die Qualität des Filmes war vergleichbar mit der der Zusammensetzung 2. Der Film, der durch die erfindungsgemäße Schaumzusammensetzung 1 aufgebracht wurde, konnte vor dem nächsten Melkvorgang leichter von Euter und Zitzen entfernt werden als der der Zusammensetzung 2.

Wenn der Verbrauch an desinfizierendem Mittel auf eine milchgebende Periode einer Kuh hochgerechnet wird, so sind anstatt ca. 3,42 kg bei durchschnittlich zwei Melkvorgängen pro Tag der Zusammensetzung 2 nur noch ca. 2,34 kg der Schaumzusammensetzung 1 bei entsprechenden zwei Melkvorgängen pro Tag notwendig.

## Patentansprüche

1. Nichttherapeutisches Verfahren zur Desinfektion von Zitzen oder Eutern von Säugetieren, wobei die Zitzen oder Euter nach dem Melkvorgang mit einer in Form eines Schaumes vorliegenden desinfizierenden Zusammensetzung behandelt werden, die folgende Bestandteile enthält:
ein Polyvinylalkohol-Polymer und/oder Copolymer und
eine Ethercarbonsäure,
einen Desinfektionswirkstoff und
ein oder mehrere Tenside als Schaumbildner und
30 bis 90 Vol-% eines Gases.

2. Verfahren nach Anspruch 1, **dadurch gekennzeichnet, daß** die genannte Ethercarbonsäure unter die allgemeine Formel R-(OC₂H₄)ₙ-OCH₂-COOH fällt, wobei R für einen Alkylrest mit 4 bis 10 C-Atomen und n für eine ganze Zahl im Bereich von 2 bis 8 steht.

3. Verfahren nach einem der Ansprüche 1 oder 2, **dadurch gekennzeichnet, daß** der genannte Desinfektionswirkstoff ausgewählt ist aus der Gruppe Polyvinylpyrrolidon-Jod, Chlorhexidin, Derivate von Chlorhexidin, Gemische aus Milchsäure und Wasserstoffperoxid oder Mischungen derselben.

4. Verfahren nach einem oder mehreren der Ansprüche 1 bis 3, **dadurch gekennzeichnet, daß** die Behandlung mit dem Schaum so erfolgt, dass der Schaum zunächst in einen Behälter gefüllt wird und dann die Zitzen oder Euter in diesen Behälter getaucht werden.

5. Verfahren nach einem oder mehreren der Ansprüche 1 bis 4, **dadurch gekennzeichnet, daß** die desinfizierende Zusammensetzung folgende Zusammensetzung aufweist:
a) 1 bis 10 Gew-% Polyvinylalkohol-Polymer oder Copolymer,
b) 0,2 bis 5 Gew-% einer Ethercarbonsäure der allgemeinen Formel R-(OC₂H₄)ₙ-OCH₂-COOH, wobei R für einen Alkylrest mit 4 bis 10 C-Atomen und n für eine ganze Zahl im Bereich von 2 bis 8 steht, als filmbildende Mittel,
c) 0,01 bis 5 Gew-% Desinfektionswirkstoff und
d) 0,1 bis 10 Gew-% Tenside.

6. Verfahren nach einem oder mehreren der Ansprüche 1 bis 5, **dadurch gekennzeichnet, dass** Polyvinylalkohol-Polymere oder Copolymere mit Molmassen im Bereich von 18.000 bis 200.000 D eingesetzt werden.

7. Verfahren nach einem oder mehreren der Ansprüche 1 bis 6, **dadurch gekennzeichnet, dass** als Desinfektionsmittel 0,02 bis 0,3 Gew-% Jod in Form von Polyvinylpyrrolidon-Jod eingesetzt wird.

8. Verfahren nach einem oder mehreren der Ansprüche 1 bis 7, **dadurch gekennzeichnet, dass** als Desinfektionswirkstoff 0,05 bis 0,5 Gew-% Chlorhexidin eingesetzt wird.

9. Verfahren nach einem oder mehreren der Ansprüche 1 bis 8, **dadurch gekennzeichnet, dass** als Tenside anionische, kationische oder nichtionische Tenside eingesetzt werden.

10. Verfahren nach Anspruch 9, **dadurch gekennzeichnet, dass** als Tenside solche ausgewählt aus der Gruppe Alkylsulfate, Alkylsulfonate, Alkylbenzolsulfonate, Fettalkoholethoxylate, Alkylglycoside oder Mischungen derselben eingesetzt werden.

11. Verfahren nach Anspruch 1, **dadurch gekennzeichnet, dass** der Schaum 40 bis 80 Vol-% eines Gases enthält.

12. Schaumzusammensetzung zur Desinfektion von Zitzen oder Eutern von Säugetieren, enthaltend:
a) 1 bis 10 Gew-% Polyvinylalkohol-Polymer und/oder Copolymer mit Molmassen im Bereich von 18.000 bis 200.000 D,
b) 0,2 bis 5 Gew-% einer Ethercarbonsäure der allgemeinen Formel R-(OC₂H₄)ₙ-OCH₂-COOH, wobei R für einen Alkylrest mit 4 bis 10 -C-Atomen und n für eine ganze Zahl im Bereich von 2 bis 8 steht, als filmbildende Mittel,
c) 0,02 bis 0,3 Gew-% Jod in Form von Polyvinylpyrrolidon-Jod oder 0,05 bis 0,5 Gew-% Chlorhexidin als Desinfektionswirkstoff und
d) 3 bis 10 Gew-% eines Tensids
e) 30 bis 90 Vol-% eines Gases.

13. Schaumzusammensetzung nach Anspruch 12, **dadurch gekennzeichnet, dass** sie 40 bis 80 Vol-% eines Gases enthält.

## Claims

1. A non-therapeutic method of disinfecting mammals' teats and udders, wherein the teats or udders are treated, after the milking operation, with a disinfecting composition in the form of a foam containing the following components:
a polyvinyl alcohol polymer and/or copolymer and
an ether carboxylic acid,
a disinfectant and
one or more surfactants as foaming agents and
30 to 90 % by volume of a gas.

2. A method according to claim 1, **characterized in that** said ether carboxylic acid falls within the general formula R-(OC₂H₄)ₙ-OCH₂-COOH, wherein R represents an alkyl radical having 4 to 10 C-atoms and n represents an integer in the range of 2 to 8.

3. A method according to either of claims 1 or 2, **characterized in that** said disinfectant is selected from the group of polyvinylpyrrolidone-iodine, chlorhexidine, chlorhexidine derivatives, mixtures of lactic acid and hydrogen peroxide or blends thereof.

4. A method according to one or more of claims 1 to 3, **characterized in that** the treatment with the foam is carried out by initially introducing the foam into a container and then dipping the teats or udders in this container.

5. A method according to one or more of claims 1 to 4, **characterized in that** the disinfecting composition has the following composition:
a) 1 to 10 % by weight polyvinyl alcohol polymer or copolymer,
b) 0.2 to 5 % by weight of an ether carboxylic acid of general formula R-(OC₂H₄)ₙ-OCH₂-COOH, wherein R represents an alkyl radical having 4 to 10 C-atoms and n represents an integer in the range of 2 to 8, as the film-forming agent,
c) 0.01 to 5 % by weight disinfectant, and
d) 0.1 to 10 % by weight surfactants.

6. A method according to one or more of claims 1 to 5, **characterized in that** polyvinyl alcohol polymers or copolymers having molar masses in the range of 18,000 to 200,000 D are used.

7. A method according to one or more of claims 1 to 6, **characterized in that** 0.02 to 0.3 % by weight of iodine in the form of polyvinylpyrrolidone-iodine is used as the disinfectant.

8. A method according to one or more of claims 1 to 7, **characterized in that** 0.05 to 0.5 % by weight of chlorhexidine is used as the disinfectant.

9. A method according to one or more of claims 1 to 8, **characterized in that** anionic, cationic or nonionic surfactants are used as surfactants.

10. A method according to claim 9, **characterized in that** the surfactants used are selected from the group of alkyl sulfates, alkyl sulfonates, alkylbenzene sulfonates, fatty alcohol ethoxylates, alkyl glycosides or blends thereof.

11. A method according to claim 1, **characterized in that** the foam contains 40 to 80 % by volume of a gas.

12. A foam composition for disinfecting mammals' teats and udders containing:
a) 1 to 10 % by weight polyvinyl alcohol polymer and/or copolymer having molar masses in the range of 18,000 to 200,000 D,
b) 0.2 to 5 % by weight of an ether carboxylic acid of general formula R-(OC₂H₄)ₙ-OCH₂-COOH, wherein R represents an alkyl radical having 4 to 10 C-atoms and n represents an integer in the range of 2 to 8, as the film-forming agent,
c) 0.02 to 0.3 % by weight iodine in the form of polyvinylpyrrolidone- iodine or 0.05 to 0.5 % by weight chlorhexidine as the disinfectant and
d) 3 to 10 % by weight of a surfactant,
e) 30 to 90 % by volume of a gas.

13. A foam composition according to claim 12, **characterized in that** it contains 40 to 80 % by volume of a gas.

## Revendications

1. Procédé non thérapeutique de désinfection de pis ou de mamelles de mammifères, les pis ou les mamelles étant traitées après le processus de traite avec une composition désinfectante se présentant sous forme d'une mousse qui contient les composants suivants :
un polymère et/ou un copolymère d'alcool de polyvinyle et
un acide d'éther carboxylique,
une substance active désinfectante et
un ou plusieurs tensioactifs comme agents moussants et
30 à 90 % en volume d'un gaz.

2. Procédé selon la revendication 1, **caractérisé en ce que** l'acide d'éther carboxylique cité correspond à la formule générale R-(OC₂H₄)ₙ-OCH₂-COOH, dans laquelle R désigne un radical alkyle comportant 4 à 10 atomes de carbone et n désigne un nombre entier de 2 à 8.

3. Procédé selon l'une des revendications 1 ou 2, **caractérisé en ce que** la substance active désinfectante citée est choisie dans le groupe comprenant l'iode de polyvinylpyrrolidone, la chlorhexidine, les dérivés de chlorhexidine, des mélanges d'acide lactique et de peroxyde d'hydrogène ou des mélanges de ceux-ci.

4. Procédé selon l'une ou plusieurs des revendications 1 à 3, **caractérisé en ce que** le traitement avec la mousse s'effectue en remplissant tout d'abord un récipient de mousse et en plongeant ensuite les pis ou les mamelles dans ce récipient.

5. Procédé selon l'une ou plusieurs des revendications 1 à 4, **caractérisé en ce que** la composition désinfectante présente la composition suivants :
a) 1 à 10 % en poids de polymère ou copolymère d'alcool de polyvinyle,
b) 0,2 à 5 % en poids d'un acide d'éther carboxylique de formule générale R-(OC₂H₄)ₙ-OCH₂-COOH, dans laquelle R désigne un radical alkyle comportant 4 à 10 atomes de carbone et n désigne un nombre entier de 2 à 8, comme agent filmogène,
c) 0,01 à 5 % en poids de la substance active désinfectante et
d) 0,1 à 10 % en poids de tensioactifs.

6. Procédé selon l'une ou plusieurs des revendications 1 à 5, **caractérisé en ce que** les polymères ou copolymères d'alcool de polyvinyle sont utilisés en une masse molaire de l'ordre de 18 000 à 200 000 D.

7. Procédé selon l'une ou plusieurs des revendications 1 à 6, **caractérisé en ce que** l'on utilise comme agent désinfectant, 0,02 à 0,3 % en poids d'iode sous la forme d'iode de polyvinylpyrrolidone.

8. Procédé selon l'une ou plusieurs des revendications 1 à 7, **caractérisé en ce que** l'on utilise comme substance active désinfectante 0,05 à 0,5 % en poids de chlorhexydine.

9. Procédé selon l'une ou plusieurs des revendications 1 à 8, **caractérisé en ce que** l'on utilise comme tensioactifs, des tensioactifs anioniques, cationiques ou non ioniques.

10. Procédé selon la revendication 9, **caractérisé en ce que** l'on utilise comme tensioactifs, des tensioactifs choisis dans le groupe comprenant des alkylsulfates, alkylsulfonates, alkylbenzène-sulfonates, éthoxylates d'alcool gras, alkylglycosides ou des mélanges de ceux-ci.

11. Procédé selon la revendication 1, **caractérisé en ce que** la mousse contient 40 à 80 % en volume d'un gaz.

12. Composition moussante pour la désinfection de pis ou de mamelles de mammifères, contenant :
a) 1 à 10 % en poids de polymère et/ou de copolymère d'alcool de polyvinyle présentant des masses molaires de l'ordre de 18 000 à 200 000 D,
b) 0,2 à 5 % en poids d'un acide d'éther carboxylique de formule générale R-(OC₂H₄)ₙ-OCH₂-COOH, dans laquelle R désigne un radical alkyle comportant 4 à 10 atomes de carbone et n désigne un nombre entier de 2 à 8, comme agent filmogène,
c) 0,02 à 0,3 % en poids d'iode sous la forme d'iode de polyvinylpyrrolidone ou 0,05 à 0,5 % en poids de chlorhexidine comme substance active désinfectante et
d) 3 à 10 % en poids d'un tensioactif,
e) 30 à 90 % en volume d'un gaz.

13. Composition moussante selon la revendication 12, **caractérisée en ce qu'**elle contient 40 à 80 % en volume d'un gaz.
